# EUROPÄISCHE PATENTSCHRIFT

(11) **EP 1 096 993 B1**
(45) Veröffentlichungstag und Bekanntmachung des Hinweises auf die Patenterteilung: **17.03.2004**
(21) Anmeldenummer: 99929215.4
(22) Anmeldetag: 15.06.1999
(51) Int. Cl.: B01F 17/00

(54) **W/O-EMULSIONSGRUNDLAGEN**
W/O EMULSION BASES
BASES D'EMULSION EAU DANS L'HUILE

(30) Priorität: 24.06.1998 DE 19828081
(43) Veröffentlichungstag der Anmeldung: 09.05.2001
(73) Patentinhaber: Cognis Deutschland GmbH & Co. KG, 40589 Düsseldorf (DE)
(72) Erfinder: BRÜNING, Stefan, D-40597 Düsseldorf (DE); ANSMANN, Achim, D-40699 Erkrath (DE); STRAUSS, Gabriele, D-40589 Düsseldorf (DE)
(86) Internationale Anmeldenummer: PCT/EP1999/004123
(87) Internationale Veröffentlichungsnummer: WO 1999/067016

(56) Entgegenhaltungen:
- DE-A- 19 509 301

## Beschreibung

Die Erfindung befindet sich auf dem Gebiet der kosmetischen Zubereitungen und betrifft W/O-Emulsionsgrundlagen mit universeller Anwendungsbreite.

### Stand der Technik

Kosmetische Zubereitungen, wie beispielsweise Cremes und Lotionen, stellen physikochemisch betrachtet Emulsionen dar, wobei man zwischen Wasser-in-Öl (W/O)- und Öl-in-Wasser (O/W)-Typen unterscheidet. Im Markt besteht ein besonderes Interesse an W/O-Emulsionen, die in ihrer Viskosität sehr unterschiedlich sein können und von festen Cremes über Softcremes bis hin zu dünnflüssigen Lotionen reichen. Bei der Herstellung dieser Zubereitungen steht dem Kosmetikchemiker eine schier unübersehbare Zahl geeigneter Ölkomponenten, Emulgatoren sowie Hilfs- und Zusatzstoffe zur Verfügung, die er in solcher Weise kombinieren muß, daß ein Produkt des gewünschten Anwendungsspektrums resultiert. Dies ist eine mitunter sehr aufwendige Tätigkeit, insbesondere dann, wenn es darum geht, Produkte mit einer definierten Viskosität herzustellen, die diese auch bei Temperaturiagerung über einen möglichst langen Zeitraum beibehalten, ohne dünnflüssig zu werden, zu vergelen oder zu separieren. Eine weitere wichtige Anforderung besteht bei der Formulierung von W/O-Emulsionen darin, die sensorischen Eigenschaften der Emulsionen im Sinne des Anwenders einzustellen. Hierbei gilt es insbesondere, Ölkomponenten unterschiedlicher Polarität und Spreitung so miteinander zu kombinieren, daß eine sogenannte "Spreitkaskade" entsteht [vgl. U.Zeidler in **Fette, Seifen, Anstrichmitt. 87, 403 (1985)** und S.Wallat et al. in **Parf.Kosm. 75, 768 (1994)].** Wegen der unterschiedlichen Polarität der Öle ist deren gemeinsame stabile Einarbeitung jedoch oft schwierig.
Die internationale Patentanmeldung **WO 95/34528** offenbart W/O-Emulsionen, die Polyolpolyhydroxystearate enthalten. Als weitere Hilfs- und Zusatzstoffe können unter anderem Co-Emulgatoren, wie beispielsweise Cirtonensäureester, Sorbitanester usw., Fette und Wachse und Stabilisatoren, wie Metallsalze von Fettsäuren zugesetzt werden.

Die komplexe Aufgabe der Erfindung hat demnach darin bestanden, Mischungen zur Verfügung zu stellen, die im Sinne eines möglichst einfachen Baukastens die Herstellung von lagerstabilen W/O-Emulsionen unterschiedlicher Viskosität erlaubt und gleichzeitig auch die Einarbeitung unterschiedlich polarer Öle gestattet.

### Beschreibung der Erfindung

Gegenstand der Erfindung sind W/O-Emulsionsgrundlagen, enthaltend
(a) Polyolpoly-12-hydroxystearate,
(b) Polyolester ausgewählt aus der Gruppe, die gebildet wird von Sorbitanester, Oligoglycerinester und Partialglyceriden,
(c) Citronensäureester,
(d) Wachskörper und
(e) Metallseifen;

Mit den Definitionen der Komponenten (a)-(e) gemäß Anspruch 1.

Überraschenderweise wurde gefunden, daß Mischungen der Komponenten (a) bis (e) unabhängig von der Polarität der damit kombinierten Ölkörper zu W/O-Emulsionen führen, die viskositäts- und lagerstabil sind. Werden die Komponenten (a) und (b+c+d+e) vorzugsweise im Gewichtsverhältnis 10 : 90 bis 30 : 70 oder 40 : 60 bis 60 : 40 abgemischt, können in Abhängigkeit des Gewichtsverhältnisses von wäßriger zu öliger Phase sowohl Cremes als auch Lotionen erhalten werden.

### Polyolpoly-12-hydroxystearate

Bei den Polyolpoly-12-hydroxystearaten, die als Komponente (a) eingesetzt werden, handelt es sich um bekannte Stoffe, die beispielsweise unter der Marke Dehymuls® PGPH vertrieben werden. In diesem Zusammenhang sei auf die internationale Patentanmeldung **WO 95/34528** (Henkel) verwiesen. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methyolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit,
(f) Zucker mit 5 bis 12 Kohlenstoffatomen, wie beispielsweise Glucose oder Saccharose;
(g) Aminozucker wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 ( 8 bis 15) Gew.-% |
| Pentaglycerine | 2 bis 10 ( 3 bis 8) Gew.-% |
| Oligoglycerine | ad 100 Gew.-%. |

### Sorbitanester

Als Gruppe geeigneter Polyolester, die die Komponente (b) bilden, kommen Sorbitanester in Frage, die Umsetzungsprodukte des Sorbitans mit Fettsäuren darstellen und der allgemeinen Formel (I) folgen, 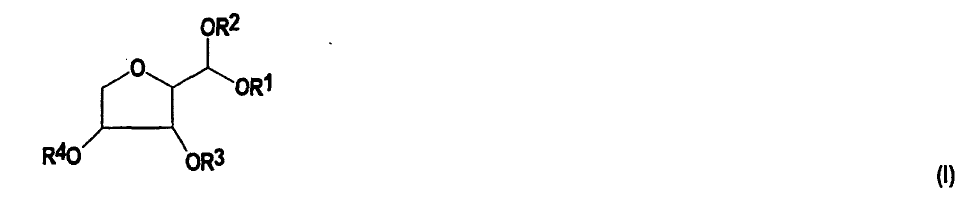 in der R¹, R², R³ und R⁴ unabhängig voneinander für Wasserstoff oder gesättigte und/oder ungesättigte, lineare oder verzweigte Acylreste mit 6 bis 22 Kohlenstoffatomen stehen, mit der Maßgabe, daß mindestens einer der Reste R¹ bis R⁴ für einen Acylrest steht. Typische Beispiele sind Sorbitanester, die sich von den folgenden Fettsäuren ableiten: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Bevorzugt sind Sorbitanester auf Basis von Fettsäuren mit 12 bis 18 Kohlenstoffatomen wie beispielsweise Laurinsäure, Kokosfettsäure, Palmitinsäure, Stearinsäure, Isostearinsäure und Ölsäure. Da das Sorbitan über vier primäre Hydroxylgruppen verfügt, die grundsätzlich alle der Veresterung zugänglich sind, stellen die Sorbitanester in der Regel Gemische von Mono-, Di- und Triestem dar; Vollester sind hingegen nur in geringen Mengen vorhanden. Bevorzugt sind sogenannte Sesquiester, d.h. Sorbitanester, die im Mittel 1,5 Estergruppen enthalten.

### Oligoglycerinester

Als weitere Gruppe geeigneter Polyolester kommen Oligoglycerinester in Frage, welche durch Kondensation von technischen Oligoglycerinen mit Fettsäuren mit 6 bis 22 und vorzugsweise 12 bis 18 Kohlenstoffatomen zugänglich sind. Die Oligoglycerinkomponente kann dabei einen Eigenkondensationsgrad von 2 bis 10 und vorzugsweise 2 bis 5 aufweisen. Besonders vorteilhaft sind Ester, die sich von technischen Diglycerinen oder Triglycerinen ableiten und mit 1 bis 6, vorzugsweise 2 bis 5 Mol - bezogen auf freie Hydroxylgruppen - linearer oder verzweigter Fettsäuren mit 12 bis 18 Kohlenstoffatomen kondensiert werden. Typische Beispiele sind Oligoglycerinester auf Basis von technischen Di-/Triglyceringemischen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Besonders bevorzugt sind Oligoglycerinester auf Basis von Palmitinsäure, Stearinsäure, Isostearinsäure sowie deren Gemischen.

### Partialglyceride

Partialglyceride, also Monoglyceride, Diglyceride und deren technische Gemische, die ebenfalls als Polyolester in Betracht kommen, können herstellungsbedingt noch geringe Mengen Triglyceride enthalten. Die Partialglyceride folgen der Formel **(II)**, 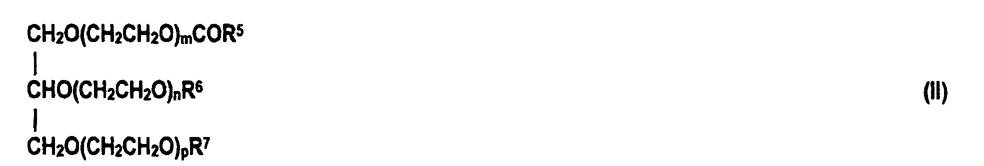 in der R⁵CO für einen linearen oder verzweigten, gesättigten und/oder ungesättigten Acylrest mit 6 bis 22, vorzugsweise 12 bis 18 Kohlenstoffatomen, R⁶ und R⁷ unabhängig voneinander für R⁵CO oder OH und die Summe (m+n+p) für 0 oder Zahlen von 1 bis 100, vorzugsweise 5 bis 25 steht, mit der Maßgabe, daß mindestens einer der beiden Reste R⁶ und R⁷ OH bedeutet. Typische Beispiele sind Monound/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

### Citronensäureester

Citronensäureester, die im Sinne der Erfindung als Komponente (c) in Frage kommen, folgen der Formel **(III)**, 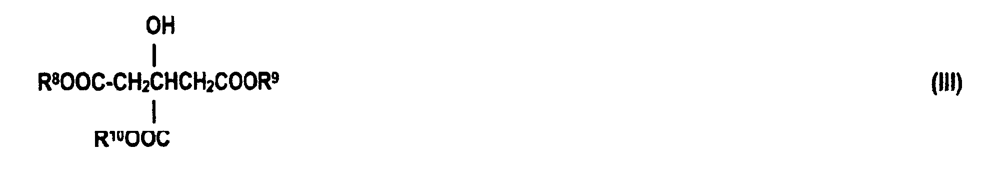 in der R⁸, R⁹ und R¹⁰ für Wasserstoff oder einen linearen oder verzweigten Alkyl- und/oder Alkenylrest und/oder den Rest eines Polyols mit 2 bis 12 Kohlenstoffatomen und 2 bis 12 vorzugsweise 3 bis 8 Hydroxylgruppen steht, mit der Maßgabe, daß mindestens einer der Reste R⁸ bis R¹⁰ von Wasserstoff verschieden ist. Typische Beispiele sind Citronensäureester, die sich von folgenden primären Alkoholen ableiten können: Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Stearyl-, Cetearyl- oder Talgfettalkohol. Des weiteren kommen als Alkoholkomponenten auch Polyole in Frage als da sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10, wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Da die Citronensäure drei Carboxylgruppen besitzt, die alle der Veresterung zugänglich sind, kann es sich bei den Citronensäureestem um Mono-, Di- oder Tricitrate sowie deren Gemische handeln. Werden als Alkohole Polyole eingesetzt, wie beispielsweise Trimethylolpropan oder Pentaerythrit, können auch verbrückte Komplexester entstehen. In einer bevorzugten Ausführungsform der Erfindung werden Citronensäureester eingesetzt, die durch gemeinsame Kondensation von Citronensäure mit Alkoholen (z.B. Kokosfettalkohol und Stearylalkohol) und Polyolen (z.B. TMP oder Pentaerythrit) erhalten werden [vgl. Deutsche Patentschrift **DE-PS 1165574** (Dehydag)].

### Wachskörper

Die Wachskörper gemäß der Erfindung sind ausgewählt aus der Gruppe der naturlichen pflanzlichen oder tierischen Wachse, der Mineralwachse, der Petrochemische Wachse und der synthetischen Hartwachse.

Unter Wachskörpem sind im Sinne der Erfindung vorzugsweise solche Stoffe zu verstehen, die
- bei 20°C knetbar, fest bis brüchig hart, grob bis feinkristallin, durchscheinend bis opak, aber nicht glasartig sind,
- oberhalb von 40°C ohne Zersetzung schmelzen sowie
- schon wenig oberhalb des Schmelzpunktes verhältnismäßig niedrigviskos sind und dabei keine Fäden ziehen.

Typische Beispiele für geeignete Wachskörper sind natürliche pflanzliche oder tierische Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin, Bürzelfett, Mineralwachse, wie z.B. Ceresin oder Ozokerit, petrochemische Wachse, wie etwa Petrolatum, Paraffin- und Mikrowachse. Weiterhin in Frage kommen auch synthetische Hartwachse, wie z.B. Montanesterwachse, Sasolwachse, hydrierte Jojobawachse, Polyalkylenwachse und Polyethylenglycolwachse. Vorzugsweise werden Bienenwachs, Lanolin oder Montanwachs eingesetzt.

### Metallseifen

Metallseifen, die als stabilisierende Komponente (e) enthalten sind, folgen vorzugsweise der Formel **(IV)**,

(R¹¹COO)ₙ-X (IV)

in der R⁸CO für einen linearen oder verzweigten, gesättigten oder ungesättigten Acylrest mit 6 bis 22, vorzugsweise 12 bis 18 Kohlenstoffatomen und X für Magnesium, Aluminium oder Zink und n für eine Zahl entsprechend der Wertigkeit von X steht. Typische Beispiele sind die entsprechenden Magnesium-Aluminium- und/oder Zinksalze der folgenden Carbönsäuren: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Ricinolsäure, 12-Hydroxystearinsäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Vorzugsweise werden Magensiumstearat, Aluminiumstearat oder Zinkstearat eingesetzt.

### W/O-Emulsionsgrundlagen

In einer bevorzugten Ausführungsform der Erfindung werden Emulsionsgrundlagen eingesetzt, die die Komponenten (a) bis (e) in den folgenden Mengenverhältnissen enthalten:
(a) 5 bis 95, vorzugsweise 25 bis 70 und insbesondere 35 bis 50 Gew.-% Polyolpoly-12-hydroxystearate,
(b) 2 bis 35, vorzugsweise 10 bis 30 und insbesondere 15 bis 25 Gew.-% Sorbitanester, Oligoglycerinester und/oder Partialglyceride,
(c) 1 bis 40, vorzugsweise 5 bis 30 und insbesondere 10 bis 20 Gew.-% Citronensäureester,
(d) 1 bis 25, vorzugsweise 5 bis 20 und insbesondere 10 bis 15 Gew.-% Wachskörper und
(e) 0,5 bis 25, vorzugsweise 5 bis 15 Gew.-% Metallseifen,
mit der Maßgabe, daß sich die Mengenangaben gegebenenfalls mit Wasser und weiteren Hilfs- und Zusatzstoffen zu 100 Gew.-% ergänzen.

### Gewerbliche Anwendbarkeit

Die erfindungsgemäßen Emulsionsgrundlagen können zur Herstellung einer Vielzahl kosmetischer oder pharmazeutischer Zubereitungen eingesetzt werden, wie beispielsweise Cremes, Lotionen, Salben und dergleichen, wobei deren Einsatzmenge üblicherweise im Bereich von 2 bis 25 und vorzugsweise 4 bis 15 Gew.-% - bezogen auf die Mittel - liegt. Die Emulsionsgrundlagen, vorzugsweise aber die Endformulierungen, können als weitere Hilfs- und Zusatzstoffe milde Tenside, Ölkörper, Co-Emulgatoren, Überfettungsmittel, Perlglanzwachse, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe in Betracht.

Als Co-Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) Alkylmono- und -oligoglycoside mit 8 bis 22 Kohlenstoffatomen im Alkylrest und deren ethoxylierte Analoga;
(3) Anlagerungsprodukte von 15 bis 60 Mol Ethylenoxid an Ricinusöl und/oder gehärtetes Ricinusöl;
(4) Polyglycerinpolyricinoleat, Polyglycerindimeratoder oder deren Gemische;
(5) Anlagerungsprodukte von 2 bis 15 Mol Ethylenoxid an Ricinusöl und/oder gehärtetes Ricinusöl;
(6) Partialester auf Basis linearer, verzweigter, ungesättigter bzw. gesättigter C_{6/22}-Fettsäuren, sowie Ricinolsäure, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(7) Mono-, Di- und Trialkylphosphate sowie Mono-, Di- und/oder Tri-PEG-alkylphosphate und deren Salze;
(8) Wollwachsalkohole;
(9) Polysiloxan-Polyalkyl-Polyether-Copolymere bzw. entsprechende Derivate;
(10) Mischester aus Pentaerythrit, Fettsäuren und Fettalkohol gemäß DE-PS 1165574 und/oder Mischester von Fettsäuren mit 6 bis 22 Kohlenstoffatomen und Methylglucose sowie
(11) Polyalkylenglycole.

Die Anlagerungsprodukte von Ethylenoxid und/oder von Propylenoxid an Fettalkohole, Fettsäuren, Alkylphenole oder an Ri-cinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl, N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lecithin sowie poly-ethoxylierte oder acylierte Lecithinderivate und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Unie Fettalkohole oder Hydroxyfettalkohole mit 12 bis 22 und vorzugsweise 16 bis 18 Kohlenstoffatomen und daneben Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar@ C-17, Jaguar@ C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AH-Säuren, Aminosäuren, Ceramide, Pseudoceramide, essentielle Öle, Pflanzenextrakte und Vitaminkomplexe zu verstehen. Als Antischuppenmittel können Climbazol, Octopirox und Zinkpyrethion eingesetzt werden. Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamilienöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Auf Basis der erfindungsgemäßen W/O-Emulsionsgrundlagen wurden die folgenden 7 Zubereitungen hergestellt: (1,6,7) Sonnenschutzlotionen, (2) Feuchtigkeitscreme, (3) Pflegelotion, (4) Pflegecreme, (5) Softcreme. Die Viskosität der Mittel wurde nach der Brookfield-Methode in einem RVF-Viskosimeter [23°C, Spindel 5, 10 Upm (Bsp. 1,3,6,7) bzw. Spindel TE mit Heliopath (Bsp.2,4,5)] sofort sowie nach Lagerung über 1 bis 2 Wochen bestimmt. Die Ergebnisse sind in Tabelle 1 zusammengefaßt.

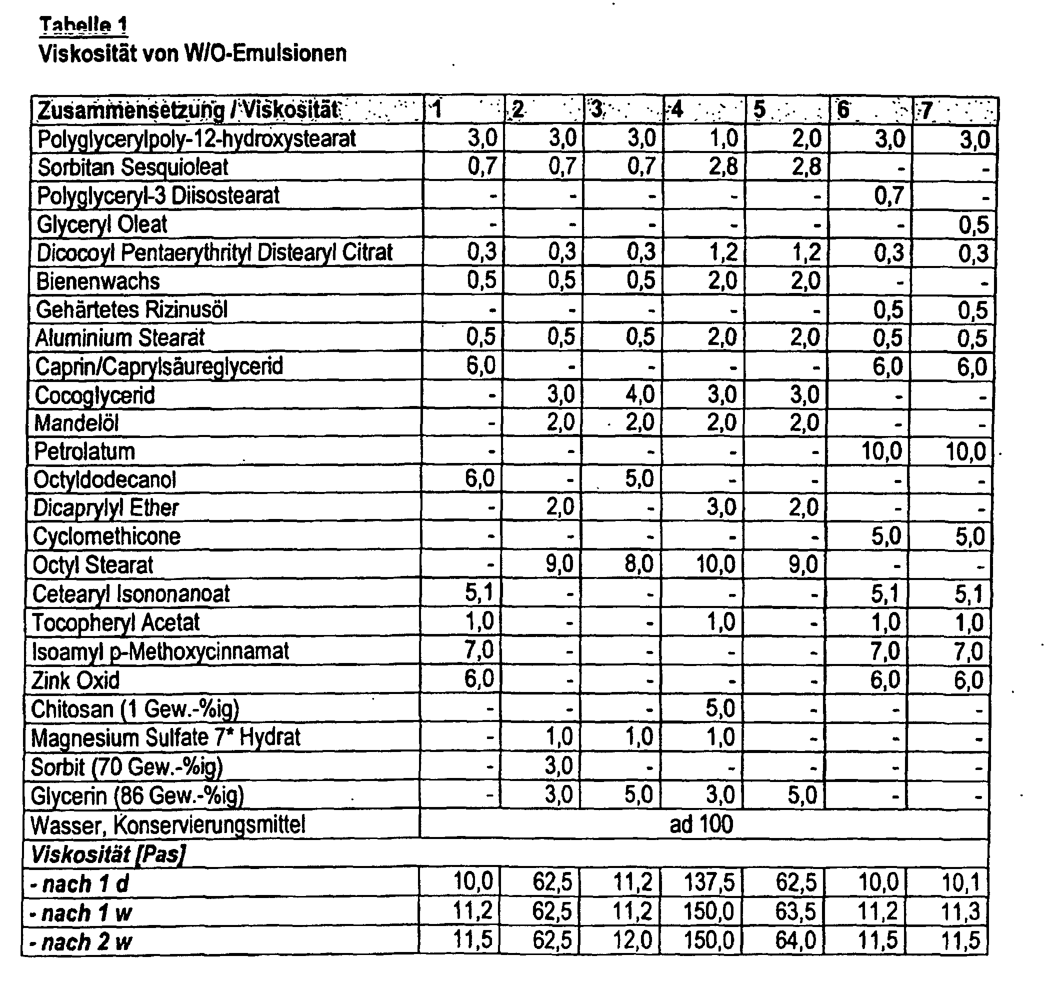

## Patentansprüche

1. W/O-Emulsionsgrundlagen, enthaltend
(a) Polyolpoly-12-hydroxystearate,
(b) Polyolester ausgewählt aus der Gruppe der Sorbitanester der Formel (I), 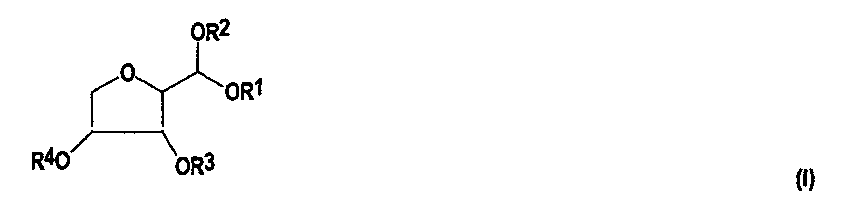 in der R¹, R², R³ und R⁴ unabhängig voneinander für Wasserstoff oder gesättigte und/oder ungesättigte, lineare oder verzweigte Acylreste mit 6 bis 22 Kohlenstoffatomen stehen, mit der Maßgabe, dass mindestens einer der Reste R¹ bis R⁴ für einen Acylrest steht; der Oligoglycerinestem von Fettsäuren mit 6 bis 22 Kohlenstoffatomen; der Partialglyceride der Formel (II) 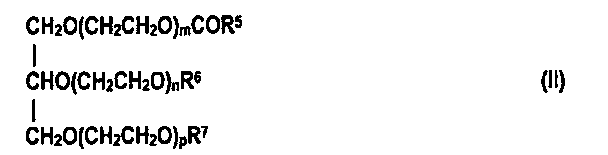 in der R⁵CO für einen linearen oder verzweigten, gesättigten und/oder ungesättigten Acylrest mit 6 bis 22 Kohlenstoffatomen, R⁶ und R⁷ unabhängig voneinander für R⁵CO oder OH und die Summe (m+n+p) für 0 oder Zahlen von 1 bis 100 steht, mit der Maßgabe, dass mindestens einer der beiden Reste R⁶ und R⁷ OH bedeutet,
(c) Citronensäureester der Formel **(III),** 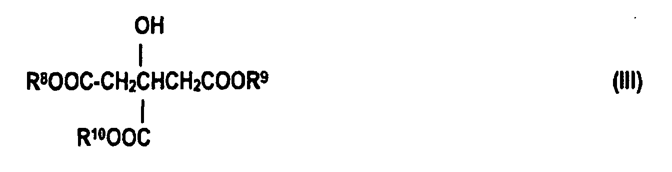 in der R8, R9 und R¹⁰ für Wasserstoff oder einen linearen oder verzweigten Alkyl- und/oder Alkenylrest und/oder den Rest eines Polyols mit 2 bis 12 Kohlenstoffatomen und 2 bis 12 Hydroxylgruppen steht, mit der Maßgabe, dass mindestens einer der Reste R⁸ bis R¹⁰ von Wasserstoff verschieden ist,
(d) Wachskörper ausgewählt aus der Gruppe der natürlichen pflanzlichen oder tierischen Wachse, der Mineralwachse, der petrochemische Wachse, und der synthetischen Hartwachse,
(e) Metallseifen.

2. W/O-Emulsionsgrundlage nach Anspruch 1, **dadurch gekennzeichnet, dass** sie als Komponente (a) Polyglycerinpoly-12-hydroxystearate enthalten.

3. W/O-Emulsionsgrundlage nach einem der Ansprüche 1 bis 2, **dadurch gekennzeichnet, dass** sie als Komponente (d) Bienenwachs, Lanolin und/oder Montanwachs enthalten.

4. W/O-Emulsionsgrundlage nach mindestens einem der Ansprüche 1 bis 3, **dadurch gekennzeichnet, dass** sie
(a) 5 bis 95 Gew.-% Polyolpoly-12-hydroxystearate,
(b) 2 bis 35 Gew.-% Sorbitanester, Oligoglycerinester und/oder Partialglyceride,
(c) 1 bis 40 Gew.-% Citronensäureester,
(d) 1 bis 25 Gew.-% Wachskörper und
(e) 0,5 bis 25 Gew.-% Metallseifen,
mit der Maßgabe enthalten, dass sich die Mengenangaben gegebenenfalls mit Wasser und weiteren Hilfs- und Zusatzstoffen zu 100 Gew.-% ergänzen.

## Claims

1. W/O emulsion bases containing
(a) polyol poly-12-polyhydroxystearates,
(b) polyol esters selected from the group consisting of sorbitan esters corresponding to formula **(I)**: 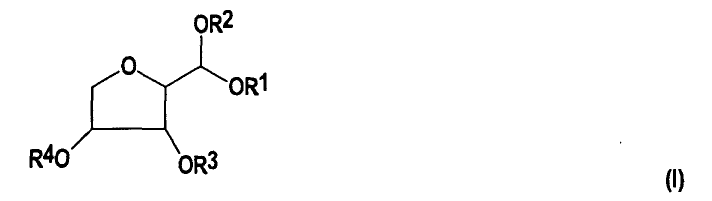 in which R¹, R², R³ and R⁴ independently of one another represent hydrogen or saturated and/or unsaturated, linear or branched acyl groups containing 6 to 22 carbon atoms, with the proviso that at least one of the substituents R¹ to R⁴ is an acyl group; oligoglycerol esters of fatty acids containing 6 to 22 carbon atoms; partial glycerides corresponding to formula **(II):** 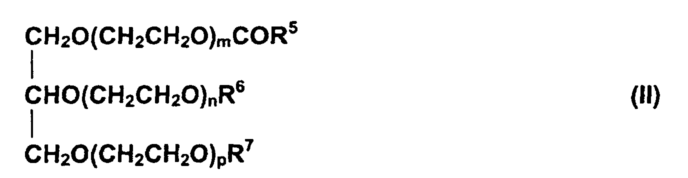 in which R⁵CO is a linear or branched, saturated and/or unsaturated acyl group containing 6 to 22 carbon atoms, R⁶ and R⁷ independently of one another have the same meaning as R⁵CO or represent OH and the sum (m+n+p) is 0 or a number of 1 to 100, with the proviso that at least one of the two substituents R⁶ and R⁷ represents OH,
(c) citric acid esters corresponding to formula (III): : 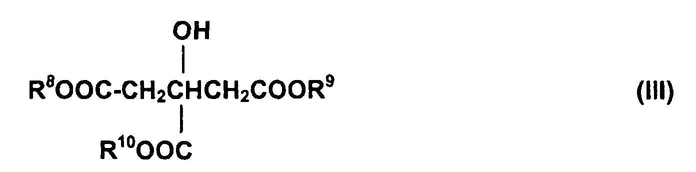 in which R⁸, R⁹ and R¹⁰ represent hydrogen or a linear or branched alkyl and/or alkenyl group and/or the residue of a polyol containing 2 to 12 carbon atoms and 2 to 12 hydroxyl groups, with the proviso that at least one of the substituents R⁸ to R¹⁰ is not hydrogen,
(d) wax components selected from the group of vegetable or animal waxes, mineral waxes, petrochemical waxes and synthetic hard waxes,
(e) metal soaps.

2. W/O emulsion bases as claimed in claim 1, **characterized in that** they contain polyglycerol poly-12-hydroxystearates as component (a).

3. W/O emulsion bases as claimed in claim 1 or 2, **characterized in that** they contain beeswax, lanolin and/or montan wax as component (d).

4. W/O emulsion bases as claimed in at least one of claims 1 to 3, **characterized in that** they contain
(a) 5 to 95% by weight of polyol poly-12-polyhydroxystearates,
(b) 2 to 35% by weight of sorbitan esters, oligoglycerol esters and partial glycerides,
(c) 1 to 40% by weight of citric acid esters,
(d) 1 to 25% by weight of wax components and
(e) 0.5 to 25% by weight of metal soaps
with the proviso that the quantities shown add up to 100% by weight, optionally with water and other auxiliaries and additives.

## Revendications

1. Substrats pour émulsions E/H (eau/huile) contenant
a) un poly 12-hydroxystéarate de polyol,
b) un ester de polyol choisi dans le groupe des esters de sorbitane de formule I 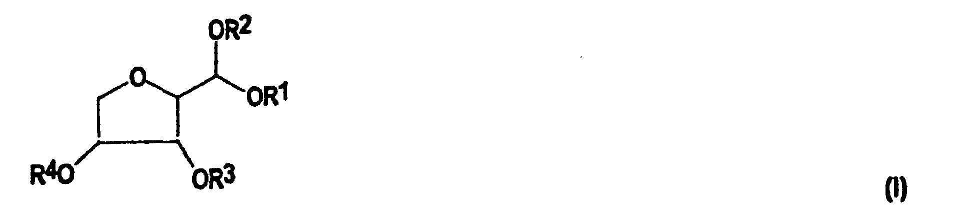 dans laquelle R¹, R², R³ et R⁴ indépendamment les uns des autres représentent de l'hydrogène ou des restes acyle saturés et/ou non saturés, linéaires ou ramifiés ayant de 6 à 22 atomes de carbone, avec la précision qu'au moins un des restes R¹ à R⁴ représente un reste acyle, des esters d'oligoglycérol d'acides gras ayant de 6 à 22 atomes de carbone. des glvcérides partiels de formule (II) 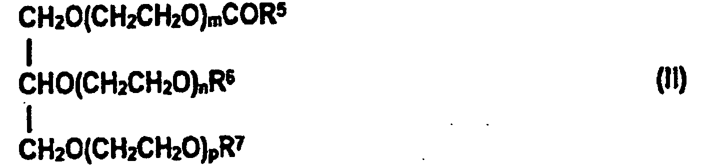 dans laquelle R⁵CO représente un reste acyle linéaire ou ramifié, saturé et/ou non saturé ayant de 6 à 22 atomes de carbone, R⁶ et R⁷ indépendamment l'un de l'autre représentent R⁵CO ou OH et
la somme (m + n + p) représente 0 ou des nombres allant de 1 à 100, avec la précision qu'au moins un des deux restes R⁶ et R⁷ signifie OH,
c) un ester d'acide citrique de formule (III) 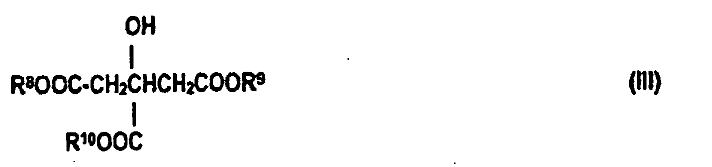 dans laquelle R⁸, R⁹ et R¹⁰ représentent de l'hydrogène ou un reste alkyle et/ou alkényle linéaire ou ramifié et/ou le reste d'un polyol ayant de 2 à 12 atomes de carbone et de 2 à 12 groupes hydroxyle avec la précision qu'au moins un des restes R⁸ à R¹⁰ est différent de l'hydrogène,
d) un composé de cire choisi dans le groupe des cires végétales ou animales naturelles, des cires minérales, des cires pétrochimiques et des cires dures synthétiques, et
e) des savons métalliques.

2. Substrats pour émulsion E/H selon la revendication 1,
**caractérisés en ce qu'**
ils renferment comme composant a) des poly 12-hydroxystéarates de polyglycérol.

3. Substrats pour émulsion E/H selon l'une des revendications 1 à 2,
**caractérisés en ce qu'**
ils renferment comme composant d) de la cire d'abeilles, de la lanoline et/ou de la cire Montana.

4. Substrats pour émulsion E/H selon au moins l'une des revendications 1 à 3,
**caractérisés en ce qu'**
ils renferment
a) de 5 à 95 % en poids de poly 12-hydroxystéarates de polyol,
b) de 2 à 35 % en poids d'ester de sorbitane, d'ester d'oligoglycérol, et/ou des glycérides partiels,
c) de 1 à 40 % en poids d'ester d'acide citrique,
d) de 1 à 25 % en poids de composé de cire et
e) de 0,5 à 25 % en poids de savons métalliques,
avec la précision que les données en quantités le cas échéant avec de l'eau et d'autres adjuvants et additifs se complètent à 100 % en poids.
